# EUROPÄISCHE PATENTSCHRIFT

(11) **EP 0 962 228 B1**
(45) Veröffentlichungstag und Bekanntmachung des Hinweises auf die Patenterteilung: **06.05.2009**
(21) Anmeldenummer: 99110257.5
(22) Anmeldetag: 27.05.1999
(51) Int. Cl.: A61M 1/36, A61M 39/20

(54) **Verfahren zum Befüllen eines Schlauchsystems mit einer Spülflüssigkeit und Schlauchsystem zur Verwendung bei dem Verfahren**
Method for filling a tubing system with a rinsing fluid and tubing system therefore
Méthode de remplissage d'un système de tubes avec un liquide de rinçage et système de tubes à cet effet

(30) Priorität: 29.05.1998 DE 19824015
(43) Veröffentlichungstag der Anmeldung: 08.12.1999
(73) Patentinhaber: Fresenius HemoCare GmbH, 61352 Bad Homburg v.d.H. (DE)
(72) Erfinder: Ludt, Claudia Dr., 66606 St. Wendel (DE); Mathieu, Bernd Dr., 66583 Spiesen (DE); Neumann, Hans-Jürgen Dr., 66606 St.Wendel (DE); Pusinelli, Thomas, 63674 Altenstadt (DE); Witt, Rüdiger Dr., 24229 Strande (DE)
(74) Vertreter: Luderschmidt, Schüler & Partner

(56) Entgegenhaltungen:
- EP-A- 0 203 513
- EP-B- 0 305 364
- WO-A-97/40869
- DE-A- 2 818 146
- GB-A- 2 168 263
- US-A- 4 215 688
- US-A- 4 227 527
- CAVANAUGH D.J., KROSNER S.P., ROLLO A.H., WEBER H.G.: "Hemodialysis blood transport system" IBM TECHNICAL DISCLOSURE BULLETIN, Bd. 19, Nr. 3, August 1976 (1976-08), Seiten 765-769, XP002124307

## Beschreibung

Die Erfindung betrifft ein Verfahren zum Befüllen eines Schlauchsystems mit einer Spülflüssigkeit unter Aufrechterhaltung der Sterilität und ein Schlauchsystem zur Verwendung bei dem Verfahren.

Bei verschiedenen akuten und chronischen Erkrankungen ist es erforderlich, eine Behandlung von Körperflüssigkeiten außerhalb des Körpers in einem extrakorporalen Kreislauf durchzuführen. Derartige Behandlungen finden bei der Hämodialyse oder Hämofiltration statt, wobei das Blut in einem Dialysator der Dialysebehandlung unterzogen wird. Neben den Hämodialyse- oder Hämofiltrationsvorrichtungen sind auch Zellseparatoren bekannt, die es erlauben, Blut des Patienten in seine Fraktionen zu trennen. Die Zellseparation erfolgt ebenfalls in einem extrakorporalen Kreislauf.

Da die Blutbehandlung unter sterilen Bedingungen durchzuführen ist, werden bei den bekannten Blutbehandlungs- oder -verarbeitungsvorrichtungen Schlauchsysteme eingesetzt, die zum einmaligen Gebrauch bestimmt sind. Zum Anschluß an den Patienten verfügen die bekannten Schlauchsysteme über eine Blutzuführleitung mit einem arteriellen und eine Blutrückführleitung mit einem venösen Patientenanschluß.

Zur Vorbereitung des extrakorporalen Kreislaufes wird der für das Blut vorgesehene Teil des Schlauchsystems mit physiologischer Kochsalzlösung gespült und möglichst luftfrei befüllt.

Bei den nicht rezirkulierenden Verfahren zum Befüllen des Schlauchsystems wird die Kochsalzlösung aus einem Behälter über die Blutzuführleitung angesaugt, wobei die Spülflüssigkeit dann durch das Schlauchsystem strömt und über die Blutrückführleitung in einen weiteren Behälter geleitet wird, der zur Aufnahme der verbrauchten Kochsalzlösung dient. Dieses Verfahren hat den Nachteil eines relativ hohen Verbrauchs an Spülflüssigkeit.

Von den nicht rezirkulierenden Verfahren zum Befüllen des Schlauchsystems unterscheiden sich die rezirkulierenden Verfahren, bei denen der Vorratsbehälter und der Auffangbehälter zusammengefaßt sind. Dadurch kann die Kochsalzlösung mehrfach durch das Schlauchsystem zirkulieren, ohne daß der Behälter wieder aufgefüllt werden muß. Zur Konnektierung der Patientenanschlüsse verfügt der Behälter über Anschlußstücke mit selbstdichtenden Septen, die von den Kanülen der Patientenanschlüsse durchstochen werden. Der Vorteil der rezirkulierenden Verfahren liegt in dem geringen Verbrauch an Spülflüssigkeit. Nachteilig ist jedoch, daß bei der Konnektierung der Patientenanschlüsse an den Behälter die Gefahr besteht, daß die Kanülen der Patientenanschlüsse beschädigt werden. Diese sollen aber beim Anstechen der Gefäße des Patienten optimal geschliffen und scharf sein. Eine medizinische Beutelanordnung zur Rezirkulation der Spülflüssigkeit ist beispielsweise aus der EP-A-0 203 513 bekannt.

Die EP-A-0 305 364 beschreibt ein Verfahren zum Befüllen eines Schlauchsystems, bei dem die Patientenanschlüsse über eine Brücke kurzgeschlossen werden, wobei die Kochsalzlösung über einen Seitenast des Schlauchsystems zugeführt wird. Die Patientenanschlüsse können bereits fabrikseitig in das Kurzschlußstück eingebracht werden, so daß das Schlauchsystem dem Anwender steril zur Verfügung steht. Nachteilig ist, daß die Spülflüssigkeit über die Patientenanschlüsse rezirkuliert. Dies führt vor allem bei den sogenannten geschlossenen Systemen, deren Nadeladapter fest mit den Kanülen verbunden sind, zu einem hohen Durchflußwiderstand. Da

Beide Patientenanschlüsse über das Kurzschlussstück miteinander verbunden sind, besteht insbesondere bei komplizierten Schlauchsystemen, wie sie bei der Zellseparation üblich sind, die Gefahr, dass sich beim Einlegen des Schlauchsystems einer der Patientenanschlüsse von dem Kurzschlussstück löst, wodurch die Sterilität verletzt wird. Des weiteren ist nachteilig, dass der eine Patientenanschluss schon dann einer unsterilen Umgebung ausgesetzt ist, wenn der andere Anschluss von dem Kurzschlussstück entfernt wird. Daher muss der Anschluss des Schlauchsystems an den Patienten sehr schnell erfolgen.

Eine Besonderheit stellen extrakorporale Schlauchsysteme mit regionaler Antikoagulation dar. Hierunter werden System verstanden, bei denen das Blut nicht innerhalb des Patienten ungerinnbar gemacht wird, sondern nur innerhalb des extrakorporalen Kreislaufs. Dies geschieht üblicherweise mit einer NatriumZitrat-Lösung, die stromab des Patientenanschlusses auf der Saugseite dem Blut zugesetzt wird. Derartige Systeme verfügen im Allgemeinen über eine sogenannte "keep-vein-open"-Funktion, die es erlaubt, das gerinnungsfähige Blut im Falle eines Stillstandes der Maschine aus den Kanülen in den Patienten zurückzuspülen, so dass eine Blutgerinnung in den Kanülen verhindert wird. Die bekannten Schlauchsysteme, bei denen eine "keep-vein-open"-Funktion realisiert ist, verfügen über Spülflüssigkeitsleitungen, die in unmittelbarer Nähe der Patientenanschlüsse in die Blutzuführ- bzw. Blutrückführleitung münden. Im Falle eines Stillstandes der Maschine wird die Kochsalzlösung aus einem Beutel über die Spülflüssigkeitsleitungen dem Schlauchsystem zugeführt.

Die US-A-4 215 688 beschreibt eine Blutbehandlungsvorrichtung mit einem Schlauchset, das eine arterielle und eine venöse Blutleitung aufweist. Zum Spülen der arteriellen Nadel wird eine Kochsalzlösung aus einem Behälter in die arterielle Blutleitung geleitet.

Der Erfindung liegt die Aufgabe zugrunde, ein Verfahren anzugeben, das die Befüllung eines Schlauchsystems mit regionaler Antikoagulation unter

Aufrechterhaltung der Sterilität erlaubt, ohne dass der Durchflusswiderstand erhöht ist und einer der Patientenanschlüsse schon dann einer unsterilen Umgebung ausgesetzt ist, wenn der andere zum Anschluss an den Patienten bereit ist.

Die Lösung dieser Aufgabe erfolgt erfindungsgemäß mit den Merkmalen des Patentanspruchs 1.

Eine weitere Aufgabe der Erfindung liegt darin, ein Schlauchsystem zur Verwendung bei dem Verfahren zur Verfügung zu stellen. Diese Aufgabe wird mit den Merkmalen des Patentanspruchs 9 bzw. 13 gelöst.

Bei dem erfindungsgemäßen Verfahren erfolgt die Rezirkulation der Spülflüssigkeit nicht über die Patientenanschlüsse, sondern die Spülflüssigkeit rezirkuliert über die Spülflüssigkeitsleitungen. Aus einem Spülflüssigkeitsbehältnis strömt die Spülflüssigkeit über die Spülflüssigkeitszuführleitung, die Blutzuführleitung, die Blutrückführleitung und die Spülflüssigkeitsrückführleitung, um dann wieder der Spülflüssigkeitszuführleitung zugeführt zu werden. Selbst wenn sich die Schutzkappe von einem der Patientenanschlüsse löst, ist der andere Patientenanschluss weiterhin einer sterilen Umgebung ausgesetzt.

Das Verfahren kann in mehreren Varianten sowie Kombinationen durchgeführt werden, die durch jeweils veränderte Schlauchsysteme ermöglicht werden. Schwerpunkte der Modifikationen sind dabei die Schutzkappen an den Patientenanschlüssen und die Leitungsführung des Schlauchsystems in der Nähe des Spülflüssigkeitsbehältnisses.

Unter Patientenanschlüssen werden nachfolgend alle Mittel verstanden, die eine Verbindung des Schlauchsystems mit den Gefäßen des Patienten erlauben. Die Patientenanschlüsse können als Nadeladapter zum Aufstecken von Kanülen ausgebildet sein, die Kanülen können aber auch bereits auf die Nadeladapter aufgesteckt oder einstückiger Bestandteil der Anschlüsse sein. Unter Schutzkappen werden nachfolgend alle Mittel verstanden, die den Patientenanschluß steril umschließen.

Damit sich die Schutzkappen während des Spülvorganges vollständig mit Spülflüssigkeit füllen, wird die in dem Leitungsabschnitt der Blutzuführ- bzw. -rückführleitung stromauf bzw. stromab der Abzweigung der Spülflüssigkeitszuführ- bzw. -rückführleitung befindliche Luft aus den Schutzkappen abgelassen. Hierzu weisen die Schutzkappen hydrophobe Membranen auf, die einen Durchtritt von Flüssigkeit verhindern, aber ein Entweichen von Luft ermöglichen.

Die venöse und arterielle Schutzkappe können als separate Bauteile ausgebildet sein, es ist aber auch möglich, daß beide Schutzkappen eine Einheit bilden. Wenn die venöse und arterielle Schutzkappe einstückig sind, können die Innenkammern der Schutzkappen unabhängig voneinander entlüftet werden, es ist aber auch möglich, daß die in der venösen Schutzkappe befindliche Luft in die arterielle Schutzkappe überführt wird.

Die Schutzkappeneinheit für ein Schlauchsystem zur Verwendung bei der einen Variante des erfindungsgemäßen Verfahrens verfügt über eine venöse und eine arterielle Innenkammer zur sterilen Aufnahme des venösen bzw. arteriellen Patientenanschlusses, wobei zumindest die venöse Innenkammer über eine hydrophobe Membran mit der Umgebung in Verbindung steht, während die Schutzkappeneinheit für ein Schlauchsystem zur Verwendung bei der anderen Variante des erfindungsgemäßen Verfahrens zwei Innenkammern aufweist, die über eine hydrophobe Membran miteinander in Verbindung stehen.

Im folgenden werden das erfindungsgemäße Verfahren sowie mehrere Ausführungsbeispiele des Schlauchsystems zur Verwendung bei dem erfindungsgemäßen Verfahren unter Bezugnahme auf die Zeichnungen näher erläutert.

Es zeigen:
- Figur 1: ein Ausführungsbeispiel eines Schlauchsystems für einen Zellseparator zusammen mit den wesentlichen Komponenten desselben in schematischer Darstellung,
- Figur 2: ein erstes Ausführungsbeispiel der die Patientenanschlüsse verschließenden Schutzkappen des Schlauchssystems von Figur 1 in schematischer Darstellung,
- Figur 3: ein weiteres Ausführungsbeispiel der Leitungsführung zur Herstellung einer Flüssigkeitsverbindung zwischen der Spülflüssigkeitszuführ- und -rückführleitung des Schlauchsystems von Figur 1 in schematischer Darstellung,
- Figur 4: ein weiteres Ausführungsbeispiel der Leitungsführung zur Herstellung der Strömungsverbindung zwischen der Spülflüssigkeitszuführ- und -rückführleitung des Schlauchsystems von Figur 1 in schematischer Darstellung,
- Figur 5: eine weiteres Ausführungsbeispiel der Leitungsführung zur Herstellung der Flüssigkeitsverbindung zwischen der Spülflüssigkeitszuführ- und -rückführleitung des Schlauchsystems von Figur 1 in schematischer Darstellung,
- Figur 6: ein weiteres Ausführungsbeispiel der die Patientenanschlüsse flüssigkeitsdicht verschließenden Schutzkappen für das Schlauchsystem von Figur 1 in schematischer Darstellung,
- Figur 7: eine Schutzkappeneinheit zur Aufnahme der Patientenanschlüsse für das Schlauchsystem von Figur 1 in schematischer Darstellung und
- Figur 8: ein weiteres Ausführungsbeispiel der Leitungsführung zur Herstellung der Strömungsverbindung zwischen der Spülflüssigkeitszuführ- und -rückführleitung und
- Figur 9: ein weiteres Ausführungsbeispiel der Schutzkappeneinheit zur Aufnahme der Patientenanschlüsse in schematischer Darstellung.

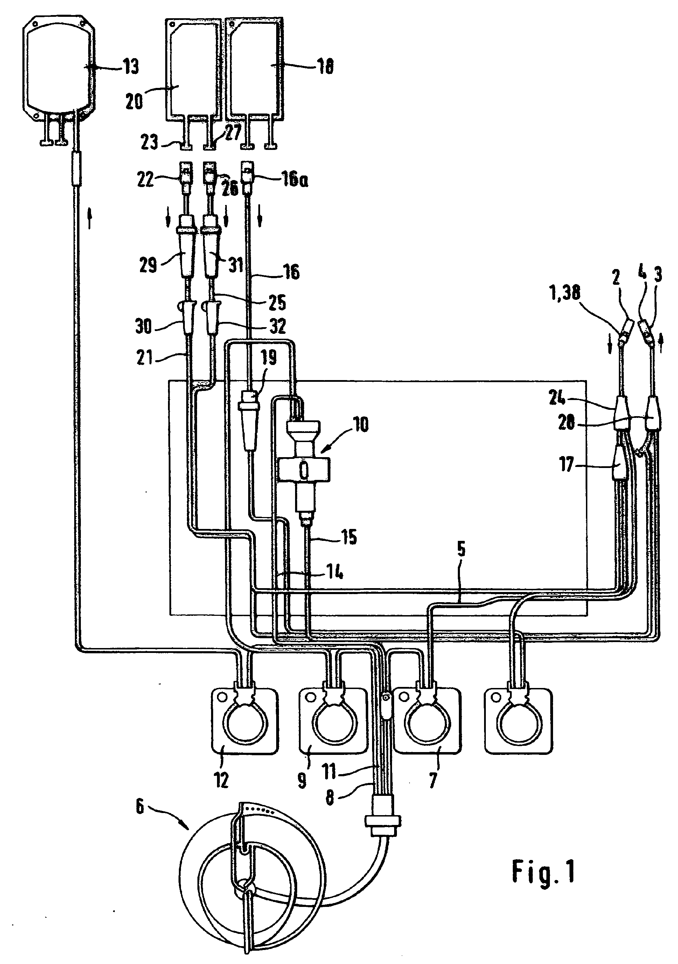

Figur 1 zeigt ein Ausführungsbeispiel eines als Thrombozyten-Set bezeichneten Schlauchsystems zusammen mit den wesentlichen Komponenten eines Zellseparators, in den sich das zur einmaligen Verwendung bestimmte Schlauchsystem einlegen läßt.

Das Schlauchsystem verfügt über einen arteriellen Patientenanschluß 1, der mit einer arteriellen Schutzkappe 2 flüssigkeitsdicht verschlossen ist und einen venösen Patientenanschluß 3, der mit einer venösen Schutzkappe 4 flüssigkeitsdicht verschlossen ist. An dem arteriellen Patientenanschluß 1 ist eine Blutzuführleitung 5 angeschlossen, die mit dem Bluteinlaß einer Separationskammer 6 verbunden ist. In die Blutzuführleitung ist eine Vollblutpumpe 7 geschaltet, die Blut des Patienten ansaugt und in die Separationskammer fördert. In der Separationskammer 6 erfolgt eine Trennung des Vollblutes in mehrere Fraktionen. Über eine an die Separationskammer 6 angeschlossene Plasmaleitung 8, in die eine Plasmapumpe 9 geschaltet ist, wird thrombozytenarmes Plasma aus der Separationskammer abgezogen. Die Plasmaleitung 8 führt zu dem Einlaß eines Luftdetektors 10. Die Thrombozyten-Fraktion wird über eine an die Separationskammer 6 angeschlossene Thrombozytenleitung 11, in die eine Zellenpumpe 12 geschaltet ist, aus der Separationskammer abgezogen und einem Transferbeutel 13 zugeführt. Die Erythrozyten-Fraktion wird über eine an die Separationskammer angeschlossene Erythrozytenleitung 14 abgezogen. Die Erythrozytenleitung 14 führt ebenfalls zu dem Einlaß des Luftdetektors 10. Der Auslaß des Luftdetektors 10 ist über eine Blutrückführleitung 15 mit dem venösen Patientenanschluß 3 verbunden.

Das Schlauchsystem verfügt ferner über eine ACD-Leitung 16, die an einer Abzweigung 24 nahe des arteriellen Patientenanschlusses 1 in die Blutzuführleitung 5 mündet. Die ACD-Leitung 16 weist an ihrem Ende einen Konnektor 16a zum Anschluß an einen ACD-Beutel 18 auf. In die ACD-Leitung ist eine Tropfkammer 19 geschaltet.

Das Schlauchset verfügt ferner über zwei Spülflüssigkeitsleitungen, die nahe des venösen bzw. arteriellen Patientenanschlusses in die Blutzuführ- bzw. - rückführleitung münden. Diese Leitungen dienen bei den bekannten Schlauchsystemen dazu, im Falle eines Stillstandes Spülflüssigkeit, insbesondere eine Kochsalzlösung aus einem Spülflüssigkeitsbeutel 20 zuzuführen, um gerinnungsfähiges Blut aus den Anschlüssen in den Patienten zurückzuspülen. Bei dem Verfahren gemäß der Erfindung dienen diese Leitungen hingegen zum Befüllen des Schlauchsystems.

Die Spülflüssigkeitszuführleitung 21, die einen Konnektor 22 zum Anschluß an einen Auslaß 23 des Beutels 20 aufweist, mündet über die Abzweigung 17 an einer Abzweigung 24 nahe des arteriellen Patientenanschlusses 1 in die Blutzuführleitung 5, während die Spülflüssigkeitsrückführleitung 25, die einen Konnektor 26 zum Anschluß an einen Einlaß 27 des Beutels 20 aufweist, an einer Abzweigung 28 nahe des venösen Patientenanschlusses 3 von der Blutrückführleitung 15 abzweigt. Stromab des Konnektors 22 ist in die Spülflüssigkeitszuführleitung eine erste Tropfkammer 29 und stromab der Tropfkammer eine erste Rollenklemme 30 geschaltet. In die Spülflüssigkeitsrückführleitung ist stromauf des Konnektors 26 eine zweite Tropfkammer 31 und stromauf der Tropfkammer eine zweite Rollenklemme 32 geschaltet.

Das Schlauchsystem kann noch weitere Komponenten umfassen, die aber der besseren Übersichtlichkeit halber nicht dargestellt sind.

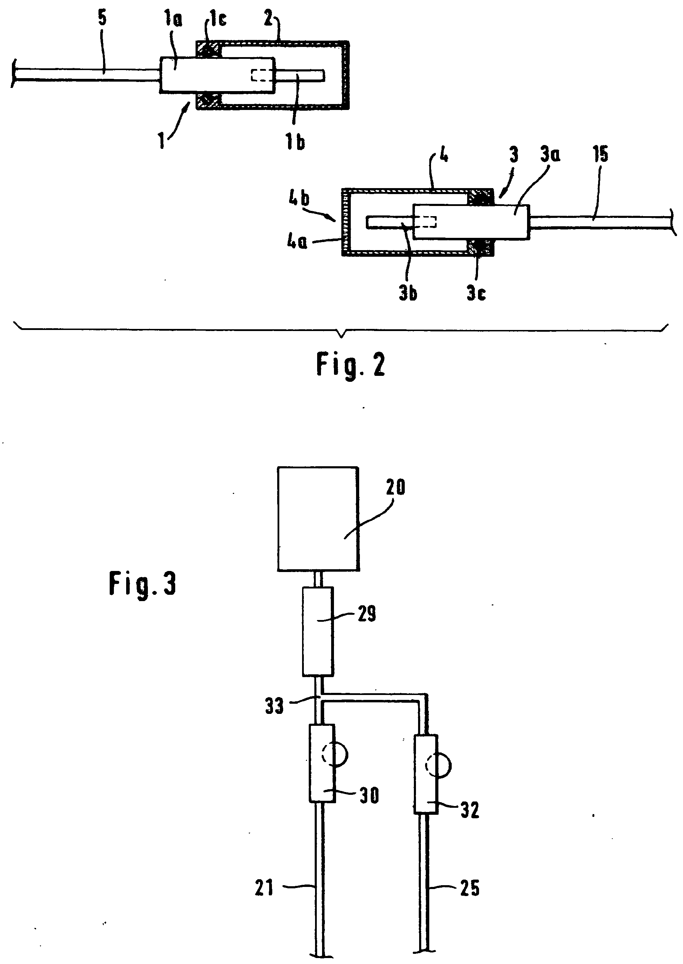

Figur 2 zeigt die Schlauchenden der Blutzuführ- und -rückführleitung 5, 15 zusammen mit den Patientenanschlüssen 1, 3, die mit den Schutzkappen 2, 4 flüssigkeitsdicht verschlossen sind. Die beiden Patientenanschlüsse 1, 3 weisen jeweils einen Nadeladapter 1a, 3a auf, an denen Kanülen 1b, 3b befestigt sind. Die auf den Anschlußstücken 1, 3 sitzenden zylindrischen Schutzkappen 2, 4 sind gegenüber den Nadeladaptern 1a, 3a mit Ringdichtungen 1c, 3c steril abgedichtet. Die venöse Schutzkappe 4 weist an ihrer Stirnseite eine mit einer hydrophoben Membran 4a, d.h. einer für Luft durchlässigen, aber für Flüssigkeit undurchlässigen Membran verschlossene Luftaustrittsöffnung 4b auf, während die arterielle Schutzkappe den arteriellen Patientenanschluß sowohl flüssigkeits- als auch luftdicht verschließt.

Das unter Bezugnahme auf die Figuren 1 und 2 beschriebene Schlauchsystem wird mit auf die Patientenanschlüsse aufgesteckten Schutzkappen als sterile Einheit bereitgestellt und wie folgt befüllt.

Das Schlauchsystem wird mit auf die Patientenanschlüsse 1, 3 aufgesetzten Schutzkappen 2, 4 in den Zellseparator eingelegt. Bei verschlossenen Rollenklemmen 30, 32 werden die Konnektoren 22, 26 der Spülflüssigkeitszuführ- bzw. -rückführleitung 21, 25 mit dem Aus- bzw. Einlaß 23, 27 des Spülflüssigkeitsbeutels 20 verbunden. Anschließend wird bei geöffneten Rollenklemmen 30, 32 die Vollblutpumpe 7 in Betrieb gesetzt, die Spülflüssigkeit aus dem Spülflüssigkeitsbeutel ansaugt. Die Spülflüssigkeit fließt durch die Spülflüssigkeitszuführleitung 21 über die Abzweigung 24 und die Blutzuführleitung 5 in die Separationskammer 6 und aus der Separationskammer 6 durch die Blutrückführleitung 15 sowie die Spülflüssigkeitsrückführleitung 25 wieder zurück in den Beutel 20. Die Spülflüssigkeit rezirkuliert dann mehrfach in dem geschlossenen Kreislauf. Die in dem Leitungsabschnitt der Blutrückführleitung 15 stromab der Abzweigung 28 befindliche Luft wird über die Hydrophobmembran 4a der venösen Schutzkappe 4 abgelassen, während sich dieser Leitungsabschnitt und die Schutzkappe mit Spülflüssigleit vollständig füllen. Nach der Beendigung des Spülvorganges werden die arterielle und venöse Schutzkappe 2, 4 von den Patientenanschlüssen 1, 3 abgenommen und die Kanülen 1b, 3b der Patientenanschlüsse werden zum Anschluß des Schlauchsystems in den Patienten gestochen.

Figur 3 zeigt die Leitungsführung im Bereich des Spülflüssigkeitsbeutels 20 einer weiteren Ausführungsform des Schlauchsystems, wobei diejenigen Teile, die denjenigen von den Figuren 1 und 2 entsprechen, mit den gleichen Bezugszeichen versehen sind. Das Ausführungsbeispiel von Figur 3 unterscheidet sich von der Ausführungsform von den Figuren 1 und 2 nur dadurch, daß die Spülflüssigkeitsrückführleitung an einem Verbindungspunkt 33 zwischen der ersten Tropfkammer 29 und der ersten Rollenklemme 30 in die Spülflüssigkeitzuführleitung 21 mündet. Eine zweite Tropfkammer entfällt bei diesem Ausführungsbeispiel. Die Spülflüssigkeit rezirkuliert über die Spülflüssigkeitszuführleitung 21, die Blutzuführleitung 5, die Blutrückführleitung 15 und die Spülflüssigkeitsrückführleitung 25. Da der Spülflüssigkeitsbeutel 20 nicht von der Spülflüssigkeit durchströmt wird, besteht keine Gefahr, daß die Spülflüssigkeit während der Rezirkulation mit Partikeln belastet wird. Gegenüber der Ausführungsform von den Figuren 1 und 2 hat das Ausführungsbeispiel von Figur 3 auch den Vorteil, daß das Schlauchsystem nach dem Befüllen sofort betriebsbereit ist. Demgegenüber muß bei dem Ausführungsbeispiel von den Figuren 1 und 2 erst ein Spiegel in der Tropfkammer 31 gesetzt werden, die während des Füllvorgangs unter Verdrängung der Luft in den Spülflüssigkeitsbeutel vollständig mit Spülflüssigkeit gefüllt wird.

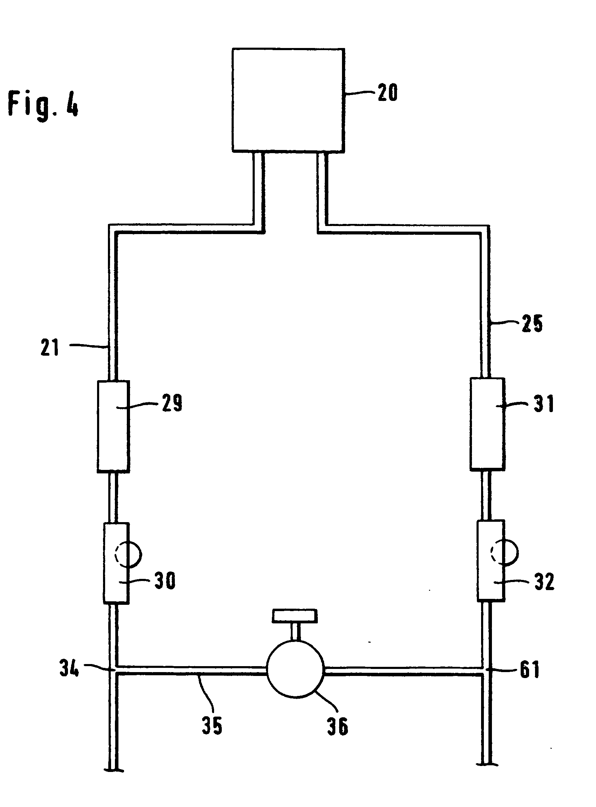

Figur 4 zeigt die Leitungsführung im Bereich des Spülflüssigkeitsbeutels einer weiteren Ausführungsform des Schlauchsystems, wobei die einander entsprechenden Teile wieder mit den gleichen Bezugszeichen versehen sind. An einem Verbindungspunkt 61 stromauf der zweiten Rollenklemme 32 zweigt von der Spülflüssigkeitsrückführleitung 25 eine Kurzschlußleitung 35 ab, die an einem Verbindungspunkt 34 stromab der ersten Rollenklemme 30 in die Spülflüssigkeitszuführleitung 21 mündet. In die Kurzschlußleitung 35 ist ein drittes Absperrorgan 36, beispielsweise eine Schlauchklemme geschaltet. Bei diesem Ausführungsbeispiel rezirkuliert die Spülflüssigkeit nicht über den Beutel 20, sondern nach Öffnen des Absperrorgans 36 durch die Kurzschlußleitung 35.

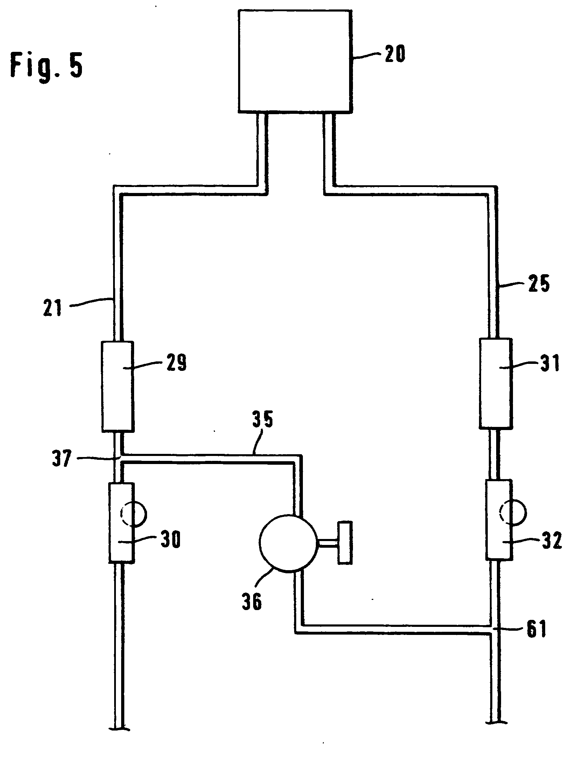

Figur 5 zeigt eine alternative Ausführungsform des Schlauchsystems mit einer Kurzschlußleitung, wobei die Teile, die denjenigen von Figur 4 entsprechen, wieder mit den gleichen Bezugszeichen versehen sind. Die an dem Verbindungspunkt 61 stromauf der zweiten Rollenklemme 32 von der Spülflüssigkeitsrückführleitung 25 abzweigende Kurzschlußleitung 35 mündet bei diesem Ausführungsbeispiel an einem Verbindungspunkt 37 zwischen der ersten Tropfkammer 29 und der ersten Rollenklemme 30 in die Spülflüssigkeitszuführleitung 21. Ansonsten entspricht die Leitungsführung derjenigen von Figur 4. Auch hier rezirkuliert die Spülflüssigkeit wieder über die Kurzschlußleitung.

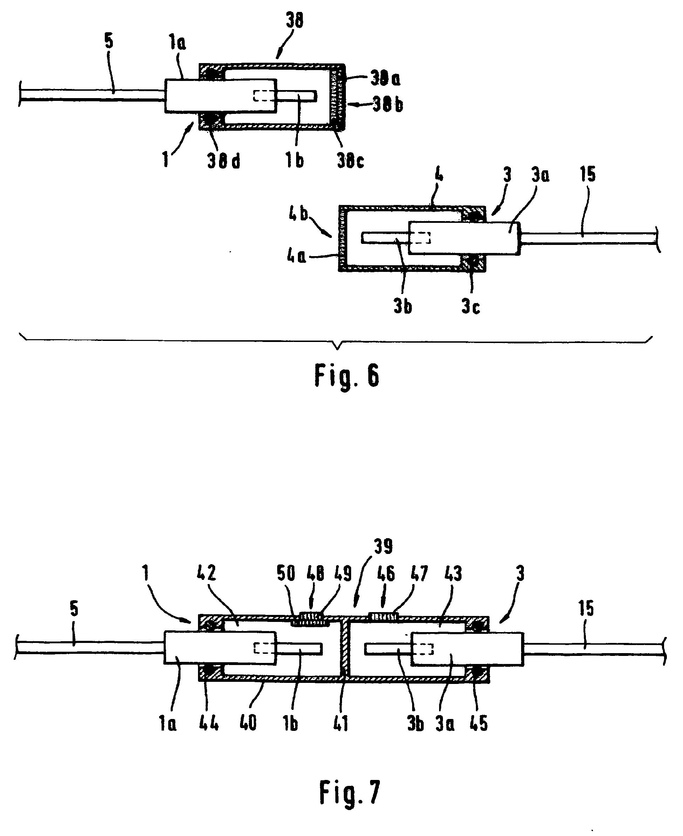

Figur 6 zeigt die venöse und arterielle Schutzkappe einer weiteren Ausführungsform des Schlauchsystems von den Figuren 1 und 2. Die Schutzkappen von Figur 6 können aber auch bei den Schlauchsystemen von den Figuren 3 bis 5 anstelle der Schutzkappen von Figur 2 Verwendung finden. Die venöse Schutzkappe 4 von Figur 6 entspricht derjenigen von Figur 2. Daher sind diese Schutzkappen auch mit den gleichen Bezugszeichen versehen. Die arterielle Schutzkappe 38 weist hingegen eine mit einer hydrophoben Membran 38a verschlossene Luftaustrittsöffnung 38b auf, wobei die Innenseite der Hydrophobmembran mit einer hydrophilen Membran 38c bedeckt ist. Die zylindrische Schutzkappe 38 ist mit einer Ringdichtung 38d flüssigkeitsdicht gegenüber dem arteriellen Patientenanschluß 1 abgedichtet.

Die beiden Schutzkappen von Figur 6 können auch einstückig sein. Figur 7 zeigt eine derartige Schutzkappeneinheit zusammen mit den venösen und arteriellen Patientenanschlüssen 1, 3 des Schlauchsystems. Die Schutzkappeneinheit 39 weist einen zylindrischen Körper 40 auf, der durch eine mittlere Trennwand 41 in eine arterielle Innenkammer 42 zur Aufnahme des arteriellen Patientenanschlusses 1 und eine venöse Innenkammer 43 zur Aufnahme des venösen Patientenanschlusses 3 getrennt ist. Gegenüber den Patientenschlüssen 1, 3 ist der zylindrische Körper 40 mit den Ringdichtungen 44, 45 flüssigkeitsdicht abgedichtet. Die venöse Innenkammer 43 steht mit der Umgebung über eine seitliche Luftaustrittsöffnung 46 in Verbindung, die mit einer hydrophoben Membran 47 verschlossen ist, während die arterielle Innenkammer 42 mit einer seitlichen Luftaustrittsöffnung 48 mit der Umgebung in Verbindung steht, die von einer hydrophoben Membran 49 verschlossen ist, deren Innenseite mit einer hydrophilen Membran 50 bedeckt ist.

Die Schutzkappen von Figur 6 und die Schutzkappeneinheit von Figur 7 haben gegenüber den Schutzkappen von Figur 2 den Vorteil, daß auch der Leitungsabschnitt der Blutzuführleitung 5 stromauf der Abzweigung 24 und die arterielle Schutzkappe 38 vollständig mit Spülflüssigkeit befüllt wird. Es können auch beide Schutzkappen mit einer Hydrophob- bzw. Hydrophilmembran bedeckt sein.

Das Verfahren zum Befüllen des Schlauchsystems mit Kurzschlußleitung (Figur 4 oder 5), dessen Patientenanschlüsse mit den Schutzkappen von Figur 6 oder der Schutzkappeneinheit von Figur 7 flüssigkeitsdicht verschlossen sind, wird nachfolgend beschrieben. Zu Beginn des Spülvorgangs wird der Spülflüssigkeitsbeutel 20 an einem erhöhten Ort angeordnet. Die erste Rollenklemme 30 in der Spülflüssigkeitszuführleitung 21 wird geöffnet, während das Absperrorgan 36 in der Kurzschlußleitung 35 und die zweite Rollenklemme 32 in der Spülflüssigkeitsrückführleitung 25 geschlossen sind. Unter dem Einfluß der Schwerkraft fließt die Spülflüssigkeit durch die Spülflüssigkeitszuführleitung 21 in die Blutzuführleitung 5. Die in dem Leitungsabschnitt der Blutzuführleitung 5 stromauf der Abzweigung 24 und in der arteriellen Schutzkappe 38 bzw. der arteriellen Innenkammer 42 der Schutzkappeneinheit 39 befindliche Luft wird dabei über die hydrophile Membran 38c bzw. 50 und die Hydrophobmembran 38a bzw. 49 aus der Schutzkappe bzw. der Schutzkappeneinheit abgelassen. Die Spülflüssigkeit füllt die arterielle Schutzkappe 38 bzw. die arterielle Innenkammer 42 der Schutzkappeneinheit 39 vollständig auf, bis sie in Kontakt mit der hydrophoben Membran kommt. Dabei wird die auf der Innenseite der Hydrophobmembran befindliche hydrophile Membran 38c bzw. 50 benetzt und damit luftundurchlässig. Erst jetzt wird die Vollblutpumpe 7 in Betrieb gesetzt und das Absperrorgan 36 in der Kurzschlußleitung 35 geöffnet, um die Befüllung des Schlauchsystems durch Rezirkulation zu ermöglichen. Die in dem Leitungsabschnitt der Blutrückführleitung 15 stromab der Abzweigung 28 und in der venösen Schutzkappe 3 bzw. der venösen Innenkammer 43 der Schutzkappeneinheit 39 befindliche Luft wird über die Hydrophobmembran 4a bzw. 47 abgelassen. Da die hydrophile Membran 38c bzw. 50 der arteriellen Schutzkappe 38 bzw. der arteriellen Innenkammer 42 der Schutzkappeneinheit 39 durch die Benetzung mit der Spülflüssigkeit luftundurchlässig geworden ist, wird verhindert, daß von der Vollblutpumpe 7 Luft in die Blutzuführleitung 5 angesaugt wird.

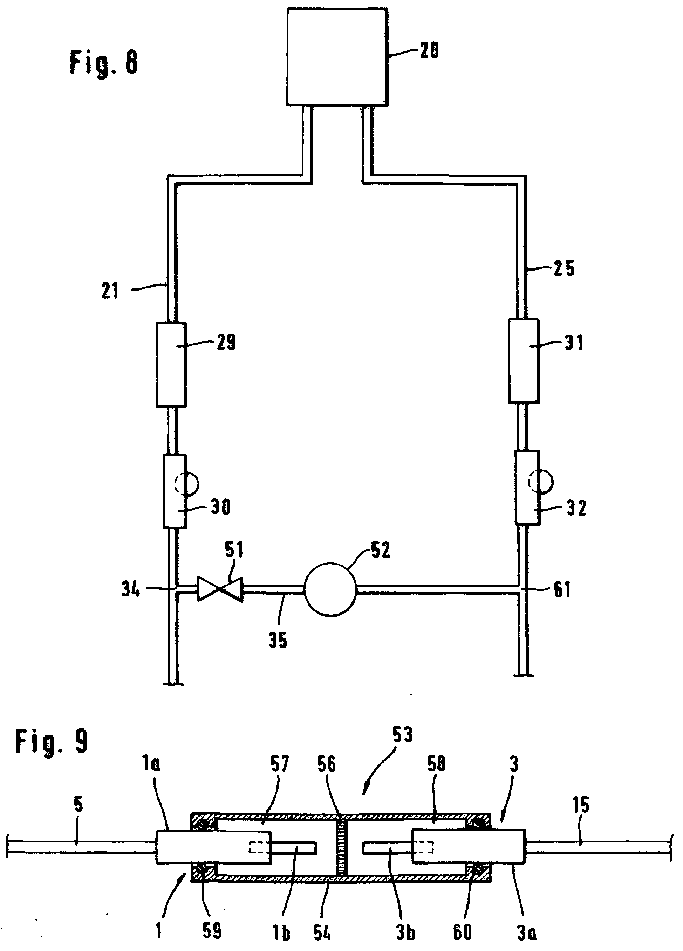

Figur 8 zeigt die Leitungsführung im Bereich des Spülflüssigkeitsbeutels einer weiteren Ausführungsform eines Schlauchsystems mit Kurzschlußleitung, das nicht die Betätigung eines Absperrorgans in der Kurzschlußleitung während des Befüllens erfordert. Diese Ausführungsform unterscheidet sich von dem Ausführungsbeispiel von Figur 4, dadurch, daß in der Kurzschlußleitung 35 anstelle des Absperrorgans 36 ein Überdruckventil 51 und zwischen dem Überdruckventil und dem Abzweigpunkt 61 ein Hydrophobfilter 52 angeordnet sind. Ansonsten entspricht die Leitungsführung derjenigen von Figur 4. Die einander entsprechenden Teile sind daher mit den gleichen Bezugsziffern versehen. Das Überdruckventil und der Hydrophobfilter können auch das Absperrorgan 36 von der Ausführungsform von Figur 5 ersetzen.

Das Überdruckventil 51 verhindert den Übertritt der Spülflüssigkeit in die Spülflüssigkeitsrückführleitung 25 sowie das Ansaugen von Luft aus dieser Leitung. Die Druckgrenze des Überdruckventils ist derart ausgelegt, daß die statische Höhe des Spülflüssigkeitsbeutels 20 nicht ausreicht, das Ventil zu öffnen, wohl aber der Systemdruck der Blutpumpe 7. Der Hydrophobfilter 52 stromauf des Überdruckventils 51 dient zum Abscheiden von in der Spülflüssigkeitsrückführleitung 25 befindlicher Luft. Wenn die Druckgrenze des Überdruckventils 51 so hoch liegt, daß die Luft durch einen in der Tropfkammer vorgesehenen Hydrophobfilter und die Hydrophobmembran in der venösen Schutzkappe sicher abgeschieden werden kann, so daß nur das relativ kleine Luftvolumen in der Spülflüssigkeitsrückführleitung 25 wieder in die Spülflüssigkeitszuführleitung 21 gelangen kann, besteht die Möglichkeit, auf den Hydrophobfilter 52 stromauf des Druckventils 51 zu verzichten.

Bei den Ausführungsformen von den Figuren 6 und 7 kann auf die hydrophile Membran 38c bzw. 50 auch verzichtet werden. Bei einem derartigen Ausführungsbeispiel ist dann zwischen dem arteriellen bzw. venösen Patientenanschluß 1 und der Abzweigung 24 in der Blutzuführleitung 5 aber ein Absperrorgan vorzusehen, das nach dem Befüllen der Blutzuführleitung unter dem Einfluß der Schwerkraft vor dem Einschalten der Blutpumpe geschlossen und erst beim Anschluß an den Patienten wieder geöffnet wird. Das Absperrorgan verhindert, daß von der Blutpumpe 7 Luft über die Hydrophobmembran 38a bzw. 49 der arteriellen Schutzkappe 38 bzw. der arteriellen Innenkammer 42 der Schutzkappeneinheit 39 angesaugt wird, so daß keine Luft in die Leitung gelangt.

Figur 9 zeigt eine weitere Ausführungsform einer Schutzkappeneinheit 53. Die Schutzkappeneinheit 53 weist einen zylindrischen Körper 54 auf, der durch eine Hydrophobmembran 56 in eine arterielle Innenkammer 57 zur Aufnahme des arteriellen Patientenanschlusses 1 und eine venöse Innenkammer 58 zur Aufnahme des venösen Patientenanschlusses 3 getrennt ist. Der zylindrische Körper 54 ist gegenüber den Patientenanschlüssen 1, 3 mit Ringdichtungen 59, 60 flüssigkeitsdicht abgedichtet.

Bei dem Ausführungsbeispiel von Figur 9 wird die venöse Innenkammer 58 der Schutzkappeneinheit 53 nach dem Einschalten der Blutpumpe 7 vollständig mit Spülflüssigkeit unter Verdrängung der darin befindlichen Luft durch die Hydrophobmembran 56 in die arterielle Innenkammer 57 befüllt. Die Hydrophobmembran 56 erlaubt zwar den Durchtritt der Luft in die Blutzuführleitung 5, die Schutzkappeneinheit wird jedoch nicht von der Spülflüssigkeit durchströmt.

## Patentansprüche

1. Verfahren zum Befüllen eines Schlauchsystems mit einer Spülflüssigkeit unter Aufrechterhaltung der Sterilität des Schlauchsystems,
wobei das Schlauchsystem aufweist:
eine Blutzuführleitung mit einem arteriellen Patientenanschluss und eine Blutrückführleitung mit einem venösen Patientenanschluss,
eine von der Blutzuführleitung stromab des arteriellen Patientenanschlusses abzweigende Spülflüssigkeitszuführleitung und eine von der Blutrückfübrleitung stromauf des venösen Patientenanschlusses abzweigende Spülflüssigkeitsrückführleitung,
mit folgenden Verfahrensschritten:
eine Flüssigkeitsverbindung zwischen der Spülflüssigkeitszuführ- und rückführleitung wird hergestellt und Spülflüssigkeit aus einem Spülflüssigkeitsbehältnis über die Flüssigkeitsverbindung zwischen der Spülflüssigkeitszuführ- und - rückführleitung wird wieder in die Spülflüssigkeitszuführleitung geleitet,
**dadurch gekennzeichnet,**
**dass** der venöse Patientenanschluss mit einer venösen Schutzkappe, die eine hydrophobe Membran aufweist, einzeln flüssigkeitsdicht verschlossen wird und der arterielle Patientenanschluss mit einer arteriellen Schutzkappe einzeln flüssigkeitsdicht verschlossen wird, wobei die venöse Schutzkappe und arterielle Schutzkappe zum Anschluss des Schlauchsystems von den Patientenanschlüssen abnehmbar sind.

2. Verfahren nach Anspruch 1, **dadurch gekennzeichnet, dass** zur Herstellung der Flüssigkeitsverbindung zwischen der Spülflüssigkeitszuführ- und -rückführleitung Spülflüssigkeit aus der Spülflüssigkeitsrückführleitung über das Spülflüssigkeitsbehältnis in die Spülflüssigkeitszuführleitung geleitet wird.

3. Verfahren nach Anspruch 1, **dadurch gekennzeichnet, dass** zur Herstellung der Flüssigkeitsverbindung zwischen der Spülflüssigkeitszuführ- und -rückführleitung Spülflüssigkeit aus der Spülflüssigkeitsrückführleitung unter Umgehung des Spülflüssigkeitsbehältnisses in die Spülflüssigkeitszuführleitung geleitet wird.

4. Verfahren nach einem der Ansprüche 1 bis 3, **dadurch gekennzeichnet, dass** die Spülflüssigkeit aus dem Spülflüssigkeitsbehältnis mit einer in die Blutzuführleitung geschalteten Blutpumpe angesaugt wird.

5. Verfahren nach einem der Ansprüche 1 bis 4, **dadurch gekennzeichnet, dass** die in dem Leitungsabschnitt der Blutrückführleitung stromab der Abzweigung der Spülflüssigkeitsrückführleitung befindliche Luft über die hydrophobe Membran der venösen Schutzkappe aus der venösen Schutzkappe abgelassen wird, so dass die venösen Schutzkappe vollständig mit Spülflüssigkeit befüllt wird.

6. Verfahren nach Anspruch 5, **dadurch gekennzeichnet, dass** die in dem Leitungsabschnitt der Blutrückführleitung stromab der Abzweigung der Spülflüssigkeitsrückführleitung befindliche Luft aus der venösen Schutzkappe unter Herstellung einer für Luft durchlässigen, aber für Flüssigkeit undurchlässigen Verbindung zwischen der venösen und der arteriellen Schutzkappe über die hydrophobe Membran der venösen Schutzkappe in die arterielle Schutzkappe überführt wird.

7. Verfahren nach Anspruch 4 oder 5, **dadurch gekennzeichnet, dass** Spülflüssigkeit unter dem Einfluß der Schwerkraft aus dem Spülflüssigkeitsbehältnis über die Spülflüssigkeitszuführleitung in die Blutzuführleitung geleitet wird, wobei die in dem Leitungsabschnitt der Blutzuführleitung stromauf der Abzweigung der Spülflüssigkeitszuführleitung befindliche Luft aus der arteriellen Schutzkappe abgelassen wird, so dass die arterielle Schutzkappe vollständig mit Spülflüssigkeit gefüllt wird und dass die Blutzuführleitung dann luftdicht verschlossen und Spülflüssigkeit mit der in die Blutzuführleitung geschalteten Blutpumpe aus dem Spülflüssigkeitsbehältnis angesaugt wird.

8. Verfahren nach Anspruch 7, **dadurch gekennzeichnet, dass** die venöse Schutzkappe durch Rezirkulation der Spülflüssigkeit vollständig mit Spülflüssigkeit gefüllt wird.

9. Schlauchsystem zur Verwendung bei dem Verfahren nach einem der Ansprüche 1 bis 8 mit
einer Blutzuführleitung (5), die einen arteriellen Patientenanschluß (1) aufweist, der mit einer arteriellen Schutzkappe (2) einzeln flüssigkeitsdicht verschlossen ist, die zum Anschluß des Schlauchsystems von dem Patientenanschluß abnehmbar ist,
einer Blutrückführleitung (15), die einen venösen Patientenanschluß (3) aufweist, der mit einer venösen Schutzkappe (4) einzeln flüssigkeitsdicht verschlossen ist, die zum Anschluß des Schlauchsystems von dem Patientenanschluß abnehmbar ist, wobei die venöse Schutzkappe (4) eine hydrophobe Membran (4a) aufweist,
einer von der Blutzuführleitung stromab des arteriellen Patientenanschlusses abzweigenden Spülflüssigkeitszuführleitung (21) und einer von der Blutrückführleitung stromauf des venösen Patientenanschlussesabzweigenden Spülflüssigkeitsrückführleitung (25), wobei die Spülflüssigkeitszuführleitung und die Spülftüssigkeitsrückführleitung an einem die Spülflüssigkeit aufnehmenden Spülflüssigkeitsbehältnis (20) angeschlossen und in die Spülflüssigkeitszuführleitung stromab des Spülflüssigkeitsbehältnisses ein erstes Absperrorgan (30) und in die Spülflüssigkeitsrückführleitung stromauf des Spülflüssigkeitsbehältnisses ein zweites Absperrorgan (32) geschaltet sind,
**dadurch gekennzeichnet, dass**
eine die Spülflüssigkeitszulührleitung (5) und Spülflüssigkeitsrückführleitung (15) verbindende Kurzschlußleitung (35) vorgesehen ist, in die ein drittes Absperrorgan (36) oder ein Überdruckventil (51) geschaltet ist.

10. Schlauchsystem nach Anspruch 9, **dadurch gekennzeichnet, dass** in der Spülflüssigkeitszuführleitung (21) stromauf des ersten Absperrorgans (30) eine erste Tropfkammer (29) und in der Spülflüssigkeitsrückführleitung (25) stromab des zweiten Absperrorgans (32) eine zweite Tropfkammer (31) angeordnet sind.

11. Schlauchsystem nach Anspruch 10, **dadurch gekennzeichnet, dass** das eine Ende der Kurzschlußleitung (35) von dem Leitungsabschnitt der Spülflüssigkeitszuführleitung (21) zwischen der ersten Tropfkammer (29) und dem ersten Absperrorgan (30) abzweigt und das andere Ende der Kurzschlußleitung von dem Leitungsabschnitt der Spülflüssigkeitsrückführleitung (25) stromauf des zweiten Absperrorgans (32) abzweigt.

12. Schlauchsystem nach Anspruch 10, **dadurch gekennzeichnet, dass** das eine Ende der Kurzschlußleitung (35) von dem Leitungsabschnitt der Spülflüssigkeitszuführleitung (21) stromab des ersten Absperrorgans (30) und das andere Ende der Kurzschlußleitung von dem Leitungsabschnitt der Spülflüssigkeitsrückfuhrleitung (25) stromauf des zweiten Absperrorgans (32) abzweigt.

13. Schlauchsystem zur Verwendung bei dem Verfahren nach einem der Ansprüche 1 bis 8 mit
einer Blutzuführleitung (5), die einen arteriellen Patientenanschluß (1) aufweist der mit einer arteriellen Schutzkappe (2) einzeln flüssigkeitsdicht verschlossen ist, die zum Anschluß des Schlauchsystems von dem Patientenanschluß abnehmbar ist,
einer Blutrückführleitung (15), die einen venösen Patientenanschluß (3) aufweist, der mit einer venösen Schutzkappe (4) einzeln flüssigkeitsdicht verschlossen ist, die zum Anschluß des Schlauchsystems von dem Patientenanschluß abnehmbar ist, wobei die venöse Schutzkappe (4) eine hydrophobe Membran (4a, 56) aufweist.
einer von der Blutzuführleitung stromab des arteriellen Patientenanschlusses abzweigenden Spülflüssigkeitszuführleitung (21) und einer von der Blutrückführleitung stromauf des venösen Patientenanschlusses abzweigenden Spülflüssigkeitsrückführleitung (25), wobei die Spülflüssigkeitzuführleitung an einem die Spülflüssigkeit aufnehmenden Spülflüssigkeitsbehältnis (20) angeschlossen und in die Spülflüssigkeitszuführleitung stromab des Spülflüssigkeitsbehältnisses ein erstes Absperrorgan (30) geschaltet ist,
**dadurch gekennzeichnet, dass**
das freie Ende der Spülflüssigkeitsrückführleitung (25) mit dem Leitungsabschnitt der Spülflüssigkeitszuführleitung zwischen dem Spülflüssigkeitsbehältnis (20) und dem ersten Absperrorgan (30) verbunden und in die Spülflüssigkeitsrückführleitung ein zweites Absperrorgan (32) geschaltet ist.

14. Schlauchsystem nach Anspruch 13, **dadurch gekennzeichnet, dass** in der Spülflüssigkeitszuführleitung (21) zwischen dem Spülflüssigkeitsbehältnis (20) und dem ersten Absperrorgan (30) eine Tropfkammer (29) angeordnet ist.

15. Schlauchsystem nach einem der Ansprüche 9 bis 14, **dadurch gekennzeichnet, dass** die arterielle Schutzkappe (1, 38) eine mit einer hydrophoben Membran (38a) verschlossene Luftaustrittsöffnung (38b) aufweist, wobei die Innenseite der hydrophoben Membran mit einer hydrophilen Membran (38c) bedeckt ist.

16. Schlauchsystem nach einem der Ansprüche 9 bis 15, **dadurch gekennzeichnet, dass** die venöse und arterielle Schutzkappe unter Bildung einer venösen und einer arteriellen Innenkammer (42,43; 57,58) einstückig sind.

17. Vorrichtung nach Anspruch 16, **dadurch gekennzeichnet, dass** die arterielle Innenkammer mit der venösen Innenkammer (57, 58) über die hydrophobe Membran (56) in Verbindung stehen.

18. Vorrichtung nach einem der Ansprüche 9 bis 18, **dadurch gekennzeichnet, dass** der venöse und/oder arterielle Patientenanschluß (1,3) eine Kanüle (1b,3b) zum Anschluß an den Patienten aufweist, die von der venösen bzw. arteriellen Schutzkappe (2,4) steril umschlossen ist.

## Claims

1. A method for filling a hose system with a rinsing fluid whilst maintaining the sterility of the hose system,
wherein the hose system comprises:
a blood supply line with an arterial patient connection and a blood return line with a venous patient connection,
a rinsing fluid supply line branching off from the blood supply line downstream of the arterial patient connection and a rinsing fluid return line branching off from the blood return line upstream of the venous patient connection,
with the following method steps:
a fluid connection between the rinsing fluid supply line and return line is produced and rinsing fluid from a rinsing fluid container is conveyed via a fluid connection between the rinsing fluid supply line and return line back into the rinsing fluid supply line,
**characterised in that**
the venous patient connection is closed individually fluid-tight with a venous protective cap, which comprises a hydrophobic membrane, and the arterial patient connection is closed individually fluid-tight with an arterial protective cap, the venous protective cap and arterial protective cap being removable from the patient connections for the connection of the hose system.

2. The method according to claim 1, **characterised in that**, in order to produce the fluid connection between the rinsing fluid supply line and return line, rinsing fluid is conveyed from the rinsing fluid return line via the rinsing fluid container into the rinsing fluid supply line.

3. The method according to claim 1, **characterised in that**, in order to produce the fluid connection between the rinsing fluid supply line and return line, rinsing fluid is conveyed from the rinsing fluid return line thereby bypassing the rinsing fluid container into the rinsing fluid supply line.

4. The method according to any one of claims 1 to 3, **characterised in that** the rinsing fluid is sucked from the rinsing fluid container by a blood pump incorporated in the blood supply line.

5. The method according to any one of claims 1 to 4, **characterised in that** the air present in the line segment of the blood return line downstream of the branching-off of the rinsing fluid return line is bled from the venous protective cap via the hydrophobic membrane of the venous protective cap, so that the venous protective cap is completely filled with rinsing fluid.

6. The method according to claim 5, **characterised in that** the air present in the line segment of the blood return line downstream of the branching-off of the rinsing fluid return line is transferred from the venous protective cap via the hydrophobic membrane of the venous protective cap into the arterial protective cap, a connection permeable to air, but impermeable to fluid, thereby being produced between the venous and the arterial protective cap.

7. The method according to claim 4 or 5, **characterised in that** rinsing fluid is conveyed under the effect of gravity from the rinsing fluid container via the rinsing fluid supply line into the blood supply line, the air present in the line segment of the blood supply line upstream of the branching-off of the rinsing fluid supply line being bled from the arterial protective cap, so that the arterial protective cap is completely filled with rinsing fluid and that the blood supply line is then closed airtight and rinsing fluid is sucked out of the rinsing fluid container with the blood pump incorporated in the blood supply line.

8. The method according to claim 7, **characterised in that** the venous protective cap is completely filled with rinsing fluid by recirculation of the rinsing fluid.

9. A hose system for use with the method according to any one of claims 1 to 8 with
a blood supply line (5), which comprises an arterial patient connection (1) which is closed individually fluid-tight with an arterial protective cap (2), which is removable from the patient connection for the connection of the hose system,
a blood return line (15), which comprises a venous patient connection (3) which is closed individually fluid-tight with a venous protective cap (4), which is removable from the patient connection for the connection to the hose system, the venous protective cap (4) having no hydrophobic membrane (4a),
a rinsing fluid supply line (21) branching off from the blood supply line downstream of the arterial patient connection and a rinsing fluid return line (25) branching off from the blood return line upstream of the venous patient connection, the rinsing fluid supply line and the rinsing fluid return line being connected to a rinsing fluid container (20) accommodating the rinsing fluid and a first shut-off element (30) being incorporated in the rinsing fluid supply line downstream of the rinsing fluid container and a second shut-off element (32) being incorporated in the rinsing fluid return line upstream of the rinsing fluid container,
**characterised in that**
a short-circuit line (35) connecting the rinsing fluid supply line (5) and rinsing fluid return line (15) is provided, into which short-circuit line a third shut-off element (36) or an overpressure valve (51) is incorporated.

10. The hose system according to claim 9, **characterised in that** a first drip chamber (29) is disposed in the rinsing fluid supply line (21) upstream of the first shut-off element (30) and a second drip chamber (31) is disposed in the rinsing fluid return line (25) downstream of the second shut-off element (32).

11. The hose system according to claim 10, **characterised in that** one end of the short-circuit line (35) branches off from the line segment of the rinsing fluid supply line (21) between the first drip chamber (29) and the first shut-off element (30) and the other end of the short-circuit line branches off from the line segment of the rinsing fluid return line (25) upstream of the second shut-off element (32).

12. The hose system according to claim 10, **characterised in that** one end of the short-circuit line (35) branches off from the line segment of the rinsing fluid supply line (21) downstream of the first shut-off element (30) and the other end of the short-circuit line branches off from the line segment of the rinsing fluid return line (25) upstream of the second shut-off element (32).

13. The hose system for use with the method according to any one of claims 1 to 8 with
a blood supply line (5), which comprises an arterial patient connection (1) which is closed individually fluid-tight with an arterial protective cap (2), which is removable from the patient connection for the connection of the hose system,
a blood return line (15), which comprises a venous patient connection (3) which is closed individually fluid-tight with a venous protective cap (4), which is removable from the patient connection for the connection of the hose system, the venous protective cap (4) having a hydrophobic membrane (4a, 56),
a rinsing fluid supply line (21) branching off from the blood supply line downstream of the arterial patient connection and a rinsing fluid return line (25) branching off from the blood return line upstream of the venous patient connection, the rinsing fluid supply line being connected to a rinsing fluid container (20) accommodating the rinsing fluid and a first shut-off element (30) being incorporated in the rinsing fluid supply line downstream of the rinsing fluid container,
**characterised in that**
the free end of the rinsing fluid return line (25) is connected to the line segment of the rinsing fluid supply line between the rinsing fluid container (20) and the first shut-off element (30) and a second shut-off element (32) is incorporated in the rinsing fluid return line.

14. The hose system according to claim 13, **characterised in that** a drip chamber (29) is disposed in the rinsing fluid supply line (21) between the rinsing fluid container (20) and the first shut-off element (30).

15. The hose system according to any one of claims 9 to 14, **characterised in that** the arterial protective cap (1, 38) comprises an air outlet opening (38b) closed with a hydrophobic membrane (38a), the inside of the hydrophobic membrane being covered with a hydrophilic membrane (38c).

16. The hose system according to any one of claims 9 to 15, **characterised in that** the venous and arterial protective cap are in one piece thereby forming a venous and an arterial inner chamber (42, 43; 57, 58).

17. A device according to claim 16, **characterised in that** the arterial inner chamber is connected to the venous inner chamber (57, 58) via the hydrophobic membrane (56).

18. The device according to any one of claims 9 to 18, **characterised in that** the venous and/or arterial patient connection (1, 3) comprises a cannula (1b, 3b) for the connection to the patient, which is enclosed in a sterile manner by the venous and respectively the arterial protective cap (2, 4).

## Revendications

1. Méthode de remplissage d'un système de tubes avec un liquide de rinçage tout en maintenant la stérilité du système de tubes,
le système de tubes comprenant
un conduit d'amenée de sang avec un raccordement patient de type artériel et un conduit de reflux de sang avec un raccordement patient de type veineux,
un conduit d'amenée de liquide de rinçage dérivant du conduit d'amenée de sang en aval du raccordement patient de type artériel et un conduit de reflux du liquide de rinçage dérivant du conduit de reflux du sang en amont du raccordement patient de type veineux,
comprenant les étapes de méthode suivantes :
un passage de liquide entre les conduits d'amenée et de reflux du liquide de rinçage est créé et le liquide de rinçage est acheminé hors du récipient à liquide de rinçage via le passage de liquide entre les conduits d'amenée et de reflux du liquide de rinçage à nouveau dans le conduit d'amenée du liquide de rinçage,
**caractérisée en ce que**
le raccordement patient de type veineux doté d'un capuchon de protection veineux qui comprend une membrane hydrophobe est fermé de façon individuelle et étanche aux liquides et le raccordement patient de type artériel doté d'un capuchon de protection artériel est fermé de façon individuelle et étanche aux liquides, le capuchon de protection veineux et le capuchon de protection artériel servant au raccordement au système de tubes pouvant être retirés des raccordements patient.

2. Méthode selon la revendication 1, **caractérisée en ce que**, pour créer le passage de liquide entre les conduits d'amenée et de reflux du liquide de rinçage, un liquide de rinçage est acheminé hors du conduit de reflux du liquide de rinçage à travers le récipient à liquide de rinçage dans le conduit d'amenée du liquide de rinçage.

3. Méthode selon la revendication 1, **caractérisée en ce que**, pour créer le passage de liquide entre les conduits d'amenée et de reflux du liquide de rinçage, un liquide de rinçage est acheminé hors du conduit de reflux du liquide de rinçage en contournant le récipient à liquide de rinçage dans le conduit d'amenée du liquide de rinçage.

4. Méthode selon l'une des revendications 1 à 3, **caractérisée en ce que** le liquide de rinçage est aspiré hors du récipient à liquide de rinçage à l'aide d'une pompe à sang montée dans le conduit d'amenée en sang.

5. Méthode selon l'une des revendications 1 à 4, **caractérisée en ce que** l'air se trouvant dans la section de conduit du conduit de reflux de sang en aval de la dérivation du conduit de reflux de liquide de rinçage est libéré via la membrane hydrophobe du capuchon de protection veineux hors du capuchon de protection veineux, de sorte que le capuchon de protection veineux est entièrement rempli de liquide de rinçage.

6. Méthode selon la revendication 5, **caractérisée en ce que** l'air se trouvant dans la section de conduit du conduite de reflux de sang en aval de la dérivation du conduit de reflux de liquide de rinçage est acheminé hors du capuchon de protection veineux via la création d'un passage perméable à l'air mais non perméable aux liquides entre les capuchons de protection veineux et artériel via la membrane hydrophobe du capuchon de protection veineux dans le capuchon de protection artériel.

7. Méthode selon la revendication 4 ou 5, **caractérisée en ce qu'**un liquide de rinçage est acheminé sous l'influence de la force de gravité hors du récipient à liquide de rinçage via le conduit d'amenée en liquide de rinçage dans le conduit d'amenée en sang, l'air se trouvant dans la section de conduit du conduit d'amenée en sang en amont de la dérivation du conduit d'amenée en liquide de rinçage est libéré du capuchon de protection artériel, de sorte que le capuchon de protection artériel est entièrement rempli de liquide de rinçage et de sorte que le conduit d'amenée en sang est alors fermé de façon étanche à l'air et le liquide de rinçage est aspiré hors du récipient à liquide de rinçage à l'aide d'une pompe à sang montée dans le conduit d'amenée en sang.

8. Méthode selon la revendication 7, **caractérisée en ce que** le capuchon de protection veineux est entièrement rempli de liquide de rinçage grâce à la recirculation du liquide de rinçage.

9. Système de tubes à utiliser dans la méthode selon l'une des revendications 1 à 8, doté
d'un conduit d'amenée de sang (5), qui comprend un raccordement patient de type artériel (1), qui est fermé de façon individuelle et étanche à l'aide d'un capuchon de protection artériel (2), qui peut être retiré du raccordement patient pour un raccordement au système de tubes,
d'un conduit de reflux de sang (15), qui comprend un raccordement patient de type veineux (3), qui est fermé de façon individuelle et étanche à l'aide d'un capuchon de protection veineux (4), qui peut être retiré du raccordement patient pour un raccordement au système de tubes, le capuchon de protection veineux (4) comprenant une membrane hydrophobe (4a),
d'un conduit d'amenée de liquide de rinçage (21) dérivant du conduit d'amenée en sang en aval du raccordement patient de type artériel et d'un conduit de reflux de liquide de rinçage. (25) dérivant du conduit de reflux de sang en amont du raccordement patient de type veineux, les conduits d'amenée et de reflux de liquide de rinçage étant raccordés à un récipient à liquide de rinçage (20) recevant le liquide de rinçage et un premier organe de blocage (30) étant monté dans le conduit d'amenée en liquide de rinçage en aval du récipient à liquide de rinçage et un deuxième organe de blocage (32) étant monté dans le conduit de reflux de liquide de rinçage en amont du récipient à liquide de rinçage,
**caractérisé en ce que**
un conduit de dérivation (35) reliant le conduit d'amenée en liquide de rinçage (5) et le conduit de reflux de liquide de rinçage (15) est prévu dans lequel un troisième organe de blocage (36) ou une valve de surpression (51) est monté.

10. Système de tubes selon la revendication 9, **caractérisé en ce qu'**une première chambre compte-gouttes (29) est disposée dans le conduit d'amenée en liquide de rinçage (21) en amont du premier organe de blocage (30) et une deuxième chambre compte-gouttes (31) est disposée dans le conduit de reflux de liquide de rinçage (25) en aval du deuxième organe de blocage (32).

11. Système de tube selon la revendication 10, **caractérisé en ce qu'**une extrémité du conduit de dérivation (35) dérive à partir de la section de conduit du conduit d'amenée en liquide de rinçage (21) entre la première chambre compte-gouttes (29) et le premier organe de blocage (30) et l'autre extrémité du conduit de dérivation dérive de la section de conduit du conduit de reflux de liquide de rinçage (25) en amont du deuxième organe de blocage (32).

12. Système de tube selon la revendication 10, **caractérisé en ce qu'**une extrémité du conduit de dérivation (35) dérive à partir de la section de conduit du conduit d'amenée en liquide de rinçage (21) en aval du premier organe de blocage (30) et l'autre extrémité du conduit de dérivation dérive de la section de conduit du conduit de reflux de liquide de rinçage (25) en amont du deuxième organe de blocage (32).

13. Système de tubes à utiliser dans la méthode selon l'une des revendications 1 à 8, doté
d'un conduit d'amenée de sang (5), qui comprend un raccordement patient de type artériel (1), qui est fermé de façon individuelle et étanche à l'aide d'un capuchon de protection artériel (2), qui peut être retiré du raccordement patient pour un raccordement au système de tubes,
d'un conduit de reflux de sang (15), qui comprend un raccordement patient de type veineux (3), qui est fermé de façon individuelle et étanche à l'aide d'un capuchon de protection veineux (4), qui peut être retiré du raccordement patient pour un raccordement au système de tubes, le capuchon de protection veineux (4) comprenant une membrane hydrophobe (4a, 56),
d'un conduit d'amenée de liquide de rinçage (21) dérivant du conduit d'amenée en sang en aval du raccordement patient de type artériel et d'un conduit de reflux de liquide de rinçage (25) dérivant du conduit de reflux de sang en amont du raccordement patient de type veineux, le conduit d'amenée en liquide de rinçage étant raccordé à un récipient à liquide de rinçage (20) recevant le liquide de rinçage et un premier organe de blocage (30) étant monté dans le conduit d'amenée en liquide de rinçage en aval du récipient à liquide de rinçage
**caractérisé en ce que**
l'extrémité libre du conduit d'amenée en liquide de rinçage (25) est reliée à la section de conduit du conduit d'amenée en liquide de rinçage entre le récipient à liquide de rinçage (20) et le premier organe de blocage (30) et un deuxième organe de blocage (32) est monté dans le conduit de reflux de liquide de rinçage.

14. Système de tubes selon la revendication 13, **caractérisé en ce qu'**une chambre compte-gouttes (29) est disposée dans le conduit d'amenée en liquide de rinçage (21) entre le récipient à liquide de rinçage (20) et le premier organe de blocage (30).

15. Système de tubes selon l'une des revendications 9 à 14, **caractérisé en ce que** le capuchon de protection artériel (1, 38) comprend un orifice de sortie d'air (38b) fermée par une membrane hydrophobe (38a), le côté intérieur de la membrane hydrophobe étant recouverte d'une membrane hydrophile (38c).

16. Système de tubes selon l'une des revendications 9 à 15, **caractérisé en ce que** les capuchons de protection veineux et artériel sont d'un seul tenant avec la formation de chambres internes veineuses et artérielles (42, 43 ; 57, 58).

17. Dispositif selon la revendication 16, **caractérisé en ce que** les chambres internes artérielles sont en communication avec les chambres internes veineuses (57, 58) via la membrane hydrophobe (56).

18. Dispositif selon l'une des revendications 9 à 18, **caractérisé en ce que** le raccordement patient de type veineux et/ou artériel (1, 3) comprend une canule (1b, 3b) pour un raccordement au patient, qui est fermée de manière stérile par les capuchons de protection veineux et/ou artériel (2, 4).
